(19) Europäisches Patentamt
European Patent Office
Office européen des brevets

(11) **EP 3 784 215 B1**

(12) **EUROPEAN PATENT SPECIFICATION**

(45) Date of publication and mention
of the grant of the patent:
**09.07.2025 Bulletin 2025/28**

(21) Application number: **19721535.3**

(22) Date of filing: **23.04.2019**

(51) International Patent Classification (IPC):
***A61K 9/107*** (2006.01)     ***A61K 9/48*** (2006.01)
***A61K 31/40*** (2006.01)

(52) Cooperative Patent Classification (CPC):
**A61K 9/107; A61K 9/1075; A61K 9/4858;
A61K 31/085; A61P 15/02; A61P 15/08**

(86) International application number:
**PCT/EP2019/025117**

(87) International publication number:
**WO 2019/206458 (31.10.2019 Gazette 2019/44)**

(54) **SOFT GEL CAPSULE COMPRISING A SELECTIVE ESTROGEN RECEPTOR MODULATOR**

WEICHGELKAPSEL MIT EINEM SELEKTIVEN ÖSTROGENREZEPTORMODULATOR

CAPSULE DE GEL MOU COMPRENANT UN MODULATEUR SELECTIF DES RECEPTEURS D'OESTROGENES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.04.2018 GR 20180100177**

(43) Date of publication of application:
**03.03.2021 Bulletin 2021/09**

(73) Proprietor: **Pharmathen S.A.
15351 Pallini Attikis (GR)**

(72) Inventors:
• **KARAVAS, Evangelos
153 51 Pallini Attikis (GR)**
• **KOUTRIS, Efthymios
153 51 Pallini Attikis (GR)**
• **SAMARA, Vasiliki
153 51 Pallini Attikis (GR)**
• **KOUTRI, Ioanna
153 51 Pallini Attikis (GR)**
• **KALASKANI, Anastasia
153 51 Pallini Attikis (GR)**
• **KAKOURIS, Andreas
153 51 Pallini Attikis (GR)**
• **MPENEKIS, Vasilis
153 51 Pallini Attikis (GR)**

(56) References cited:
**WO-A1-2017/149392    US-A1- 2017 333 394**

**Description**

TECHNICAL FIELD OF INVENTION

The present invention relates to a method of manufacturing of an immediate release oral liquid formulation comprising ospemifene. Also relates to a self-emulsifying drug delivery system, a self-mircoemulsifying drug delivery system and a self-nanoemulsifying drug delivery system for oral administration comprising ospemifene and manufacturing methods thereof. Interestingly, the formulations presented in the invention avoid the need for food intake by the patient in need of such treatment previously required for increased bioavailability of Ospemifene tablet formulations.

BACKGROUND OF THE INVENTION

During menopause the decline in circulating estrogen can result in structural changes, including thinning of the vaginal epithelium and atrophy of the vulva, vagina and urinary tract. The condition known as vulvovaginal atrophy can affect up to 60% postmenopausal women. Symptoms can include vaginal dryness, burning, itching, irritation and dyspareunia. Usual treatment included the use of over-the-counter lubricants and moisturizers for relief of the symptoms and the use of topical hormonal therapy such as estrogen creams, suppositories and rings to women with no other menopausal symptoms.

As the use of estrogen-based therapies have been declining over the last ten years the use of selective estrogen receptor modulators (SERMs) has been increasing. SERMs have anti-estrogenic or estrogenic effects depending on the tissue. While tamoxifen and toremifene are well known for reducing the risk of breast cancer recurrence, they have been associated they have been associated with increased risk of endometrial cancer. Raloxifene has been used for treatment and prevention of postmenopausal osteoporosis and prevention of invasive breast cancer in postmenopausal women at high risk.

Ospemifene is the first non-hormonal SERM that has been approved for the treatment of dyspareunia associated with vulvar and vaginal atrophy in postmenopausal women.

Ospemifene acts similarly to estrogens on the vaginal epithelium, building vaginal wall thickness which in turn reduces the pain associated with moderate to severe dyspareunia. Uniquely, among the approved SERMs ospemifene exhibits a strong, beneficial estrogenic effect on the vaginal epithelium and that observation led to the use of ospemifene in an immediate release form for the treatment of dyspareunia rather than postmenopausal osteoporosis and breast cancer prevention which were the originally targeted indications. The approval of ospemifene for this indication represents a significant milestone in postmenopausal women's health.

Ospemifene is a triphenylethylene, with a similar structure to tamoxifene and toremifene and one of the main metabolites of toremifene (WO96/07402 and WO97/32574). The compound is also known as (deaminohydroxy)toremifene and FC-1271a.

Ospemifene is a highly lipophilic compound and has very low solubility in aqueous medium. Following administration, ospemifene reaches peak median serum concentration in approximately two hours in fasting postmenopausal women. Interestingly, it has been found that bioavailability of ospemifene increased two- to three-fold with food intake, hence it is recommended to be administered with food as described in European patent EP1713458.

Oral administration is the most popular route for systemic effects due to its ease of ingestion, pain avoidance, versatility and most importantly patient compliance. Immediate release liquid dosage forms have also been introduced for treating patients introducing emulsions, microemulsions, self-nanoemulsifying drug delivery system (SNEDDS), self-microemulsifying drug delivery system (SMEDDS) or self-emulsifying drug delivery system (SEDDS). The microemulsions and the self-emulsifying systems enhance oral bioavailability of poorly water soluble drugs as they increase the absorption of lipophilic drugs. In addition, SMEDDS can be administrated without connection with food intake.

WO2011/82384 is related to a SEDDS or SMEDDS or SNEDDS formulation for drug delivery of a lipophilic therapeutic agent, providing enhanced modulation of solubility, stability, absorption, metabolism, and/or pharmacokinetic profile of the therapeutic agent by formulation with a lipophilic surfactant, a hydrophilic surfactant, one or more solubilizers and, optionally, digestible oils, resulting in higher bioavailability of the therapeutic agent administered to a subject in need of such therapeutic agent. WO2010/103402 describes compositions comprising a fatty acid oil mixture and at least one surfactant and methods of use thereof. The invention describes a self-nanoemulsifying drug delivery system (SNEDDS), self-microemulsifying drug delivery system (SMEDDS) or self-emulsifying drug delivery system (SEDDS) comprising a pharmaceutical pre-concentrate in the form of a gelatin capsule.

US patent US6284268 describes a self-emulsifying microemulsion or emulsion pre-concentrate containing an omega-3 fatty acid oil and poorly water soluble therapeutic agent, such as cyclosporine for oral administration.

US2017/333394 discloses self-nanoemulsifying drug delivery systems (SNEDDS) comprising a selective estrogen receptor modulator (SERM) such as ospemifene and excipients comprising Captex 8000 (glyceryl tricaprylate) as oil, PEG 200 and propylene glycol as co-surfactants and Cremophor EL as surfactant.

There is still the need to provide a formulation of ospemifene having optimized dissolution drug release profile that can be administrated without connection to food intake.

SUMMARY OF THE INVENTION

It is, therefore, an object of the present invention to provide a pharmaceutical formulation comprising Ospemifene or pharmaceutical acceptable salts or prodrug thereof that eliminates the food effect.

A further object of the present invention is to provide an immediate release formulation of self-nanoemulsifying drug delivery system (SNEDDS), self-microemulsifying drug delivery system (SMEDDS), or self-emulsifying drug delivery system (SEDDS) comprising ospemifene in a soft gelatin capsule, which is suitable for administration under fasted state.

The main objective of the present invention is to provide a proper qualitative and quantitative liquid or semisolid oral composition of Ospemifene or pharmaceutical acceptable salt thereof, prepared by a suitable manufacturing process in order to obtain a stable and efficacious dosage form with good physicochemical characteristics. Other objects and advantages of the present invention will become apparent to those skilled in the art in view of the following detailed description.

BRIEF DESCRIPTION OF THE DRAWINGS

Figure 1: Dissolution profiles for prepared ospemifene (60 mg/mL) soft gel capsules in three formulations containing different oily phase in fasted state.

Figure 2: Dissolution profiles for prepared ospemifene (60 mg/mL) soft gel capsules containing different oily phase in fasted state in comparison with Osphena in fasted and non-fasted state.

DETAILED DESCRIPTION OF THE INVENTION

The invention is defined by the scope of the appended claims.

Microemulsions are isotropic, thermodynamically stable transparent or translucent systems of oil, water and surfactant, frequently in combination with a co-surfactant with a droplet size usually in the range of 20-200 nm. They can be classified as oil-in-water, water-in-oil or bicontinuous systems depending on their structure and are characterized by ultra-low interfacial tension between oil and water phases. In addition to oral and intravenous delivery, they are amenable for sustained and targeted delivery through ophthalmic, dental, pulmonary, vaginal and topical routes. Lipid-based formulations have attracted great deal of attention to improve the oral bioavailability of poorly water soluble drugs. The most favored approach is to incorporate lipophilic drugs into inert lipid vehicles such as oils, surfactant dispersion, microemulsions, self-emulsifying formulations, self-microemulsifying formulations and liposomes.

A self-micro emulsifying drug delivery system (SMEDDS) is a drug delivery system that uses a micro-emulsion achieved by chemical rather than mechanical means. Microemulsions have significant potential for use in drug delivery, and SMEDDS are the best of these systems. SMEDDS are of particular value in increasing the absorption of lipophilic drugs taken orally. A typical SMEDDS comprises oil, hydrophilic surfactant and a co-solvent. Mutual miscibility of these excipients is important for producing a stable liquid formulation. Self-emulsification depends on the type of oil and surfactant pair, their ratios, the surfactant concentration and the temperature. SMEDDS spread readily in the GI tract and the digestive motility of the stomach and the intestine provide the agitation necessary for self-emulsification. SMEDDS can be encapsulated in hard or soft gelatin capsules. The advantages of such drug delivery systems are the improvement in poor drug solubility, the enhancement of oral bioavailability, the protection of unstable drugs against environmental conditions and a long self-life. Additionally, SMEDDS as a thermodynamically stable system can be easily stored, while the size of the droplets of the microemulsions formed by the SMEDDS generally ranges between 2 and 100 nm. As a result, the total surface area for absorption and dispersion is significantly larger than that of a solid dosage form; therefore it can easily penetrate the GI and be absorbed leading to improved bioavailability. Moreover, SMEDDS offer numerous delivery options like hard or soft gelatin capsules. Furthermore, SMEDDS are easy to manufacture and scale-up due to very simple and economical manufacturing facilities, like simple mixer with agitator and volumetric liquid filling equipment for large scale manufacturing. Also, as mentioned before, the performance of SMEDDS is independent of food. During the formulation of a SMEDDS the solubility of the drug in different oil, surfactants and co-solvents, the selection of oil, surfactant and co-solvent based on the solubility of the drug, the preparation of the phase diagram and the preparation of SMEDDS formulation by dissolving the drug in a mixture of oil, surfactant and co-solvent must be considered.

The addition of a drug to a SMEDDS is critical because the drug interferes with the self-emulsification process, which leads to a change in the optimal oil-surfactant ratio. So, the design of an optimal SMEDDS requires pre-formulation solubility and phasediagram studies.

The main excipients for the formulation of SMEDDS consist of the oil phase, the surfactants and the co-surfactants. The main criterion for selecting the oil phase is that the drug should have high solubility in it. This will minimize the volume of the formulation to deliver the therapeutic dose of the drug in the encapsulated form. The surfactant must be able to lower the interfacial tension which facilitates dispersion process during the preparation of the microemulsion, provide a flexible film that can readily deform around the droplets and be of the appropriate lipophilic character to provide the correct curvature at the interfacial region. The presence of co-surfactants allows the interfacial film sufficient flexibility to take up different curvatures required to form microemulsion over a wide range of composition.

In one embodiment the present invention provides a liquid immediate release pharmaceutical formulation, which comprises soft gelatin capsule matrix containing an effective amount of an active pharmaceutical ingredient (API) or a pharmaceutically acceptable salt, in combination with the oil phase, surfactants and co-surfactants in a specific ratio. The formulation is a self-microemulsifying drug delivery system (SMEDDS) which can be administered in fed state or in fasted state.

The term "liquid formulation" refers to an insoluble in water, lipophilic mixture of the drug compound, oil phase, surfactant and co-surfactant in a suitable carrier.

The term "prodrug" refers to compound that, after administration, is metabolized into a pharmacologically active drug. Prodrugs are pharmacologically inactive and can be metabolized into an active form within the body.

By "immediate release", it is meant a dosage form in which the active ingredient disintegrates rapidly after the administration with enhanced rate of dissolution to release the medicaments. This term excludes formulations which are adapted to provide for "modified", "controlled", "sustained", "prolonged" and "extended" release of drug.

The self-emulsifying process depends on the nature of the oil-surfactant pair, the surfactant concentration and the temperature at which self-emulsification occurs. The mechanism by which self-emulsification occurs is not yet well understood. It has been suggested by Reiss that self-emulsification takes place when the entropy change favouring dispersion is greater than the energy required to increase the surface area of the dispersion. The free energy of a conventional emulsion formulation is a direct function of the energy required to create a new surface between the two phases (oil and water phase) an can be described by

$$\Delta G = \sum_i N_i \pi r_i^2 \sigma$$

where G is the free energy associated with the process, N is the number of droplets, r is the radius of globules and $\sigma$ is the interfacial energy.

Oils can solubilize the lipophilic drug in a specific amount. It is the most important excipient because it can facilitate self-emulsification and increase the fraction of lipophilic drug transported via the intestinal lymphatic system, thereby increasing absorption from the GI tract. Long-chain triglyceride and medium-chain triglyceride oils with different degrees of saturation have been used in the design of SMEDDSs.

The oily phase, according to the present invention can comprise triglycerides like oleic acid, castor oil and ethyl oleate, Labrafac Lipophile®, fatty acid esters like isopropyl myristate, and monoglycerides like Dubcare GPE 810®. Labrafac Lipophile® is a medium chain triglyceride, used as a vehicle in oral emulsions and SMEDDS. In a preferred embodiment, the composition of said triglyceride comprises or alternatively consists of: caproic acid C6: 2% or below, e.g. below 0,1%; caprylic acid C8: 50 to 80%, e.g. 59.2%; capric acid C10: 20 to 50%, e.g. 40.5%; lauric acid C10: 3% or below, e.g. 0.2%; myristic acid C14: 1 % or below, e.g. below 0.1%.

Dubcare GPE 810® is a hydrosoluble relipidant solubilzer also used in development of microemulsion systems, which possesses excellent solvent properties. Dubcare GPE 810® is a mixture of monoesters, diesters, and triesters of glycerol and monoesters and diesters of polyethylene glycols with a mean relative molecular weight between 200-400. Optimization of the formulation during development, surprisingly revealed that Dubcare GPE 810 induced further and quicker self-emulsification of the composition and was responsible for faster and complete drug release. The oily phase according to the present invention is in an amount of from 5 to 30% by weight.

Non-ionic surfactants with high hydrophilic-lipophilic balance (HLB) values are used in formulation of SMEDDSs. The usual surfactant strength ranges between 30-60% w/w of the formulation in order to form a stable SMEDDS. Surfactants have a high HLB and hydrophilicity, which assists the rapid spreading of the formulation in the aqueous media. Surfactants are amphiphilic in nature and they can dissolve or solubilize relatively high amounts of hydrophobic drug compounds. This can prevent precipitation of the drug within the GI lumen and prolong the existence of drug molecules. The present formulation contains a reduced amount of surfactant, thereby minimizing any GI side effects. The surfactant according to the present invention is in an amount of from 10 to 50% by weight.

The selected surfactants are preferably non-ionic with low toxicity. polysorbates such as Tween 20® or Tween 80®, both are hydrophilic non-ionic surfactants and emulsifying agents that improves oral bioavailability. Other non-ionic surfactants are Cremophor®, tyloxapol and sesame oil. Cremophor® aids in solubilization and emulsification in the preparation of pharmaceutical emulsions and self-emulsifying drug delivery systems. Additionally, Miglyol® 812 and polyethylene glycol are surfactants that enhance dissolution characteristics and work as emulsifying agents.

The liquid oral formulation of the present invention further comprises a co-surfactant. Co-surfactants like diethylene glycol monoethyl ether (DEGEE, Transcutol®), propylene glycol, polyethylene glycol, may help to dissolve large amounts of hydrophilic surfactants or the hydrophobic drug in the lipid base. These solvents sometimes play the role of the co-surfactants in the microemulsion systems. The co-surfactant according to the present invention is in an amount of from 25 to 50% by weight.

Transcutol® has good solvent properties for poorly water soluble drugs, enhances drug penetration, permeation and it is used as a co-surfactant in the formulation of SMEDDS. Propylene glycol (PG) and polyethylene glycol (PEG) are surfactants, organic solvents, suitable for oral administration that may help to dissolve large amounts of either the hydrophilic surfactant or the drug in the lipid base and can act as co-surfactant in the self-emulsifying drug delivery systems.

After administration of the liquid self-emulsifying formulations, microemulsion droplets form and subsequently disperse in the GI tract to reach sites of absorption. However, if irreversible phase separation of the microemulsion occurs, an improvement of drug absorption cannot be expected. This can be avoided with the addition of a small quantity of 1 to 5% by weight of HPMC (type 2208 Ph.Eur.) to the formulation of the present invention to prevent precipitation of the drug and to increase its viscosity due to the increase in polymer concentration.

In order to investigate drug solubility in SMEDDS, excess amount of drug was added to few mL of each excipient, placed in small bottles and the mixture was vortexed, in room temperature to facilitate solubilization.

Several parameters influence the final properties of SMEDDS. Major variables that take part in the determination of the SMEDDS's properties include the surfactant/co-surfactant (S/CS) ratio and the percentage of oil (% oil). The following Table 1 shows the aforementioned oils, surfactants and co-surfactants. The percentages were defined as the ratios of oil over the surfactant/co-surfactant (S/CS) ratio. The oil/(S/CS) ratio ranges between 1/9, 2/8, 3/7, 5/5, 7/3 and 8/2 (oil amount from 5 to 30%), while the S/CS ratio ranges between 1/2, 1/1 and 2/1 (surfactant amount from 10 to 50%).

Table 1: Oils, surfactants and co-surfactants comprised in the liquid oral formulation

| Oils | Surfactants | Co-surfactants |
|---|---|---|
| Oleic acid | Tween 20® | Transcutol® |
| Isopropyl myristate | Cremophor® | Propylene glycol |
| Ethyl oleate | Tyloxapol | Polyethylene glycol |
| Dubcare GPE 810® | Miglyol 812® | |
| Castor oil | Sesame oil | |
| Labrafac lipophile® | Tween 80® | |

The solubility of the active pharmaceutical agent in each component (oil, surfactant and co-surfactant) of the formulation is presented in Table 2. The solubility is calculated by the API quantity dissolved in 5mL of each excipient.

Table 2: Solubility of the active pharmaceutical ingredient in each excipient used in the liquid oral formulation

| Excipient | Solubility (g/mL) |
|---|---|
| Tween 20® | 0.15 |
| Tween 80® | 0.10 |
| Tyloxapol | 0.14 |
| Miglyol 812 | 0.06 |
| Dubcare GPE 810® | 0.18 |
| Propylene glycol | 0.05 |
| Castor oil #1 | 0.06 |
| Castor oil #2 | 0.06 |
| Sesame oil | 0.06 |
| **PG** 200 | 0.10 |

(continued)

| Excipient | Solubility (g/mL) |
|---|---|
| **PG** 600 | 0.16 |
| Transcutol® | 0.28 |
| Ethyl Oleate | 0.02 |
| Kolliphpor® RH40 | 0.18 |
| Oleic acid | 0.01 |
| Isopropyl myristate | 0.15 |
| Labrafac Lipophile® | 0.10 |

Better solubility was exhibited in Tween 20®, Transcutol®, Tyloxapol, DubGPE810, Labrafac Lipophile®, Isopropyl myristate and PG 200.

The solubility of the API in the surfactant/co-surfactant dyadic system is given in Table 3. Tween 80® and Tween 20® were used as surfactants, Transcutol® along with PG 200 and PEG 600 were used as co-surfactants.

Table 3: Solubility of the API in the surfactant/co-surfactant dyadic system

| Ratio S/CS | Solubility (g/mL) |
|---|---|
| Tween 80®/PEG 600 | |
| 2:1 | 0.14 |
| 1:1 | 0.14 |
| 1:2 | 0.12 |
| Tween 20®/PG 600 | |
| 2:1 | 0.14 |
| 1:1 | 0.14 |
| 1:2 | 0.14 |
| Tween 20®/Transcutol® | |
| 2:1 | 0.19 |
| 1:1 | 0.21 |
| 1:2 | 0.24 |
| Tween 20®/PG 200 | |
| 2:1 | 0.12 |
| 1:1 | 0.11 |
| 1:2 | 0.12 |

Transcutol® as a co-surfactant showed the best solubility with Tween 20® as surfactants, while both polysorbates (Tween 20®/Tween 80®) paired with PEG 600 exhibited the next best solubility.

The preliminary solubility studies showed that Dubcare GPE 810®, Isopropyl myristate and Labrafac Lipophile® are suitable for the oil phase, while Tween 20® Tween 80®, PEG600 and Transcutol® are suitable for the surfactant/co-surfactant phase respectively. However, isopropyl myristate while it's been widely used in cosmetics and topical pharmaceutical formulations as a penetration enhancer such as transdermal, otic, topical, and vaginal formulations, it is not recommended for oral use therefore not a suitable candidate. In addition, Transcutol® is qualified for topical and transdermal routes of administration only and therefore cannot be selected for oral use either.

The solubility of Dubcare GPR 810® in different oil/(S/CS) was tested. The selected ratios regarding the solubility study are 1/9, 2/8, 3/7, 7/3 and 8/2 and their respective solubility values are presented in Table 4. The S/CS ration was kept constant at 1/1.

Table 4: Solubility of the API in the oil/(S/CS) ternary system

| Ratio oil/(S/CS) | Solubility (g/mL) |
|---|---|
| Dubcare GPE 810® /(Tween 80®/PEG 600) | |
| 1:9 | 0.16 |
| 2:8 | 0.15 |
| 3:7 | 0.15 |
| 7:3 | 0.08 |
| 8:2 | 0.06 |

The solubility results showed that the ratio 1/9 demonstrated the best behavior, while the ratios 2/8 and 3/7 demonstrated the next best behavior. The various ospemifene immediate release formulations of the present invention were prepared in soft gel capsules incorporating the solubility results for the components selection.

The process for preparing the SMEDDS formulation of the present invention comprises the following steps:

- surfactant and co-surfactant mixed with ratio that ranges between 1/1 and 1/2 (surfactant amount from 10 to 50%);
- the oil was added to the mixture of surfactant and co-surfactant and mixed
- the active ingredient was added to the oil/(surfactant/co-surfactant) mixture and mixed;
- the concentration of ospemifene in the final formulation was 60 mg/mL and the oil/(S/CS) ratio ranges between 1/9, 2/8, 3/7 (oil amount from 5 to 30%);
- the liquid formulation was stirred for appropriate time under slightly warming prior to the injection of the liquid formulation in soft gel capsules.

EXAMPLES

Initially, the preparation of an immediate release soft gel capsule formulation with constant release profile was evaluated using Dubcare GPE 810® and Labrafac Lipophile® as the oil phase and Tween 20®/PEG 600 or KolliphorRH40/-PEG 600 as the S/CS. Table 5 summarizes the compositions of the tested soft gel capsules.

Table 5: Composition of liquid ospemifene SNEDDS where the amount ospemifene is constant (60 mg/mL)

| Formulation | Ospemifene strength 60 mg/mL | |
|---|---|---|
| F1 Ingredients | mg | % core |
| Ospemifene | 60 | 5.2 |
| Dubcare GPE 810® | 105 | 9.0 |
| Tween 20® | 495 | 42.5 |
| PEG 600® | 504 | 43.3 |
| Total | 1164 | 100.0 |
| | | |
| F2 Ingredients | mg | % core |
| Ospemifene | 60 | 5.2 |
| Labrafac Lipohile® | 96 | 8.3 |
| Tween 20® | 495 | 42.9 |
| PEG 600® | 504 | 43.6 |
| Total | 1155 | 100.0 |
| | | |
| F3 Ingredients | mg | % core |
| Ospemifene | 60 | 5.3 |
| Dubcare GPE 810® | 315 | 28.0 |

(continued)

| **F3** Ingredients | mg | % core |
|---|---|---|
| Kolliphor RH40® | 416 | 36.9 |
| PEG 600® | 336 | 29.8 |
| Total | 1127 | 100.0 |

All formulations were prepared according to the process as previously described. All three formulations formed clear Grade A emulsions (Grade A: Rapidly forming emulsion with a clear or blueish appearance within 1 min), therefore ospemifene release from soft gel capsules was evaluated using apparatus II (rotating paddle method) of the USP 2 on a dissolution tester. The test for the soft gel capsules was performed at $37 \pm 0.5$°C with a rotation speed of 50 rpm using as dissolution medium 1000 mL of buffer solution at pH 5.0 for 120 mins (FeSSIF state) and pH 6.5 for 120 mins (FaSSIF state).

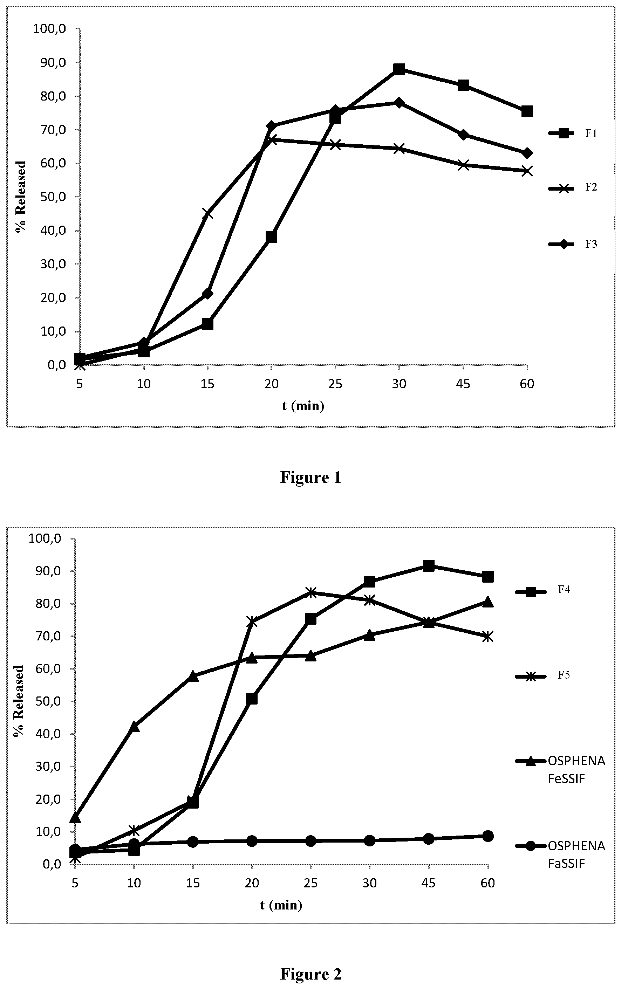

Figure 1 exhibits the in-vitro drug release of the active ingredient in fasted (FaSSIF). The comparison of the dissolution profiles showed that the Labrafac Lipophile® formulation showed the lowest drug release in the fasted state. The Dubcare GPE 810® on the other hand showed the highest drug release rate in the fasted state.

Formulation 1 (F1) contains Tween 20® as a surfactant at a percentage of 42.5% of the liquid pre-concentrate total weight. This amount however, exceeds the specified amount for daily dosage for oral administration by an individual as set by the FDA. Further optimization of the formulation includes changing the ratio of the oil to S/SC to enhance drug release.

Table 6 presents formulations F4 and F5 after optimization of the respective ratios regarding oil and S/SC phases in each composition. The dissolution rates of formulations F4 and F5 are shown in Figure 2 along with dissolution rates for Osphena® in fasted and non-fasted states.

Table 6: Optimized compositions of liquid ospemifene SNEDDS formulations (60 mg/mL)

| **Formulation** | **Ospemifene strength 60 mg/mL** | |
|---|---|---|
| **F4** Ingredients | mg | % core |
| Ospemifene | 60 | 5.7 |
| Dubcare GPE 810® | 105 | 9.9 |
| Tween 80® | 418 | 39.8 |
| PEG600 | 470.4 | 44.6 |
| Total | 1053.4 | 100.0 |
|  |  |  |
| **F5** Ingredients | mg | % core |
| Ospemifene | 60 | 5.8 |
| Dubcare GPE 810® | 262.5 | 25.8 |
| Kolliphor RH40® | 395.2 | 38.8 |
| PEG600 | 302.4 | 29.6 |
| Total | 1020.1 | 100 |

The dissolution profile of formulation F5 is similar to that of formulation F3 (as shown in Figure 1) and is indicative of an unchangeable release rate despite the change in the oil/(S/CS) ratio. On the other hand, formulation F4 showed a slightly better release rate.

Zeta potential is used to identify the charge of the droplets. The high zeta potential provides stability of dispersion and prevents aggregation. The increase in electrostatic repulsive forces between the nanoemulasion droplets prevents the coalescence of them, while a decrease of those forces causes phase separation. The SMEDDS formulations with zeta potential greater than $\pm 30$ mV are characterized as stable. Zeta potential of samples was measured by Zetasizer (Malvern instrument Nano series). The droplet size of the emulsion determines the absorption and bioavailability of the drug substance. The smaller droplets provide a larger surface tension, leading to faster drug release into aqueous medium, such as GI fluids. The average particle size of the samples was measured by Zetasizer (Malvern instrument Nano series) and is shown in Table 7.

Table 7: Zeta potential and zeta size results for the F4 composition

| F4 | | | |
|---|---|---|---|
| Zeta size (nm) | D(10) | D(50) | D(90) |
| | 7.42 | 11.6 | 21.9 |
| | | | |
| Zeta potential (mV) | -53.5 | | |

The viscosity of the liquid SNEDDS is useful to assess its ability to be filled in hard or soft gelatin capsules. If the system has very low viscosity, it may exhibit leakage from the capsule while the system with too high viscosity may create problem in pourability. The viscosity was determined by using rheometer (Brookfield Engineering Labs, model LVDV-1). The viscosity of the F4 composition was found $239\pm4.7$ cP with spindle 18, at 60 rpm and 47.8% torque at 25°C.

The prepared soft gel capsules were evaluated for long term ($25\pm2$°C and $60\pm5$% RH, for 6 months), intermediate ($30\pm2$°C and $65\pm5$% RH for 6 months), zone IV ($30\pm2$°C and $75\pm5$% RH for 6 months) and accelerated ($40\pm2$°C and $75\pm5$% for 6 months) storage conditions. Formulation F4 was found chemically stable under all those conditions without increase of unwanted impurities.

## Claims

1. A liquid self emulsifying oral drug formulation comprising a therapeutically active amount of ospemifene or a pharmaceutically acceptable salt thereof in combination with an oil PEG-8 caprylic/capric glyceride, further comprising Polysorbate 80 as a surfactant and polyethylene glycol 600 as a co-surfactant wherein the surfactant to co-surfactant ratio is from 1:9 to 8:2.

2. The oral drug formulation according to claim 1 wherein said formulation is a microemulsion or a nanoemulsion.

3. The oral drug formulation according to claim 1 wherein the dosage form is a formulation encapsulated in a soft capsule.

4. The oral drug formulation according to claim 1 wherein the oil to surfactant plus co-surfactant- ratio is from 1:2 to 2:1.

5. The oral drug formulation according to claim 1, wherein the oil phase is in the amount of 10 to 30% of the total weight of the formulation.

6. A process for preparing an oral drug formulation comprising Ospemifene or a pharmaceutically acceptable salt thereof in combination PEG-8 caprylic/capric glyceride as the oil; Polysorbate 80 as the surfactant and polyethylene glycol 600 as the co-surfactant wherein the surfactant to co-surfactant ratio is from 1:9 to 8:2, comprising the following steps:

   • Mixing surfactant and co-surfactant;
   • Adding the oil and mixing;
   • Adding Ospemifene to the oil/(surfactant/co-surfactant) and mixing,
   • Stirring the liquid formulation for appropriate time under slightly warming
   • Injecting the liquid formulation in soft gel capsules.

7. The oral drug formulation according to claim 1 for use in a method of treatment of vaginal dryness or sexual dysfunction in women during or after menopause.

## Patentansprüche

1. Eine flüssige, selbstemulgierende orale Arzneimittelformulierung, die eine therapeutisch wirksame Menge von Ospemifen oder eines pharmazeutisch verwendbaren Salzes davon in Kombination mit einem Öl PEG-8-Capryl-/Caprinsäureglycerid und darüber hinaus Polysorbat 80 als Tensid und Polyethylenglykol 600 als Co-Tensid enthält, **dadurch gekennzeichnet, dass** das Verhältnis von Tensid zu Co-Tensid zwischen 1:9 und 8:2 liegt.

2. Orale Arzneimittelformulierung nach Anspruch 1, **dadurch gekennzeichnet, dass** die o.g. Formulierung eine Mikroemulsion oder eine Nanoemulsion ist.

3. Orale Arzneimittelformulierung nach Anspruch 1, **dadurch gekennzeichnet, dass** die Darreichungsform eine in einer Weichkapsel eingekapselte Formulierung ist.

4. Orale Arzneimittelformulierung nach Anspruch 1, **dadurch gekennzeichnet, dass** das Verhältnis von Öl zu Tensid plus Co-Tensid zwischen 1:2 und 2:1 liegt.

5. Orale Arzneimittelformulierung nach Anspruch 1, **dadurch gekennzeichnet, dass** die Ölphase 10 bis 30 % des Gesamtgewichts der Formulierung beträgt.

6. Verfahren für die Zubereitung einer oralen Arzneimittelformulierung bestehend aus Ospemifen oder einem pharmazeutisch verwendbaren Salz davon in Kombination mit PEG-8-Capryl-/Caprinsäureglycerid als Öl, Polysorbat 80 als Tensid und Polyethylenglykol 600 als Co-Tensid, **dadurch gekennzeichnet, dass** das Verhältnis von Tensid zu Co-Tensid zwischen 1:9 und 8:2 liegt, welches aus den nachstehenden Schritten besteht:

   • Tensid und Co-Tensid mischen;
   • Öl hinzufügen und weitermischen;
   • Ospemifen zu dem Öl/(Tensid/Co-Tensid) hinzufügen und mischen;
   • Rühren der flüssigen Formulierung für eine angemessene Zeit unter leichtem Erwärmen;
   • Die flüssige Formulierung in Weichgelkapseln injizieren.

7. Orale Arzneimittelformulierung nach Anspruch 1 zur Verwendung als Behandlungsmethode gegen vaginale Trockenheit oder sexuelle Dysfunktion bei Frauen während oder nach der Menopause.

**Revendications**

1. Formulation pharmaceutique orale auto-émulsionnable liquide comprenant une quantité thérapeutiquement active d'ospémifène ou d'un sel pharmaceutiquement acceptable de celui-ci en combinaison avec une huile PEG-8 caprylique/glycéride caprique, comprenant en outre du polysorbate 80 en tant que tensioactif et du polyéthylène glycol 600 en tant que co-tensioactif, dans laquelle le rapport tensioactif/co-tensioactif est de 1:9 à 8:2.

2. Formulation médicamenteuse orale selon la 1ère revendication, dans laquelle ladite formulation est une microémulsion ou une nanoémulsion.

3. Formulation pharmaceutique orale selon la 1ère revendication, dans laquelle la forme posologique est une formulation encapsulée dans une capsule molle.

4. Formulation pharmaceutique orale selon la 1ère revendication, dans laquelle le rapport huile/tensioactif plus cotensioactif est de 1:2 à 2:1.

5. Formulation pharmaceutique orale selon la 1ère revendication, dans laquelle la phase huileuse est en quantité de 10 à 30 % du poids total de la formulation.

6. Procédé de préparation d'une formulation pharmaceutique orale comprenant de l'ospémifène ou un sel pharmaceutiquement acceptable de celui-ci en combinaison avec le PEG-8 caprylique/caprique glycérides en tant qu'huile ; du polysorbate 80 en tant que tensioactif et du polyéthylène glycol 600 en tant que co-tensioactif, dans lequel le rapport tensioactif/co-tensioactif est de 1:9 à 8:2, comprenant les étapes suivantes :

   • Mélanger le tensioactif et le co-tensioactif ;
   • Ajouter l'huile et mélanger ;
   • Ajouter l'ospémifène à l'huile/(tensioactif/co-tensioactif) et mélanger,
   • Remuer la formulation liquide pendant le temps approprié sous un léger réchauffement
   • Injecter la formulation liquide dans des gélules molles.

7. Formulation pharmaceutique orale selon la 1ère revendication pour une utilisation dans une méthode de traitement de la sécheresse vaginale ou du dysfonctionnement sexuel chez les femmes pendant ou après la ménopause.

**Figure 1**

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9607402 A
- WO 9732574 A
- EP 1713458 A
- WO 201182384 A
- WO 2010103402 A
- US 6284268 B
- US 2017333394 A